# EUROPEAN PATENT APPLICATION

(11) **EP 2 309 271 A1**
(43) Date of publication of application: **13.04.2011**
(21) Application number: 09305899.8
(22) Date of filing: 25.09.2009
(51) Int. Cl.: G01N 33/574

(54) **Methods for predicting the responsiveness of a patient affected with a tumor to a treatment with a tyrosine kinase inhibitor**

(71) Applicant: INSERM (Institut National de la Santé et de la Recherche Medicale), 75013 Paris (FR)
(72) Inventor: The designation of the inventor has not yet been filed
(74) Representative: Hirsch, Denise Marie

(57) **Abstract**

The present invention relates to a method for predicting the responsiveness of a patient affected with a tumor to a treatment with a tyrosine kinase inhibitor such as sunitinib. More specifically, the method of the invention comprises a step of determining the expression level of one or two markers selected in the group consisting of the VEGF₁₂₁ or VEGF₁₆₅ isoforms in a biological sample obtained from said patient.

## Description

### FIELD OF THE INVENTION:

The present invention relates to a method for predicting the responsiveness of a patient affected with a tumor to a treatment with a tyrosine kinase inhibitor (TKI), such as sunitinib.

### BACKGROUND OF THE INVENTION:

Angiogenesis is a well-known process involved in tumor growth and metastasis since such diseases are known to be associated with deregulated angiogenesis. Among proangiogenic factors, vascular endothelial growth factor (VEGF) is the mainstay of this process (2). VEGF has five main isoforms produced by alternative splicing of a single gene located on 6p21.3: VEGF₁₂₁, VEGF₁₆₅, VEGF₁₈₉, VEGF₁₄₅, and VEGF₂₀₆, which differ in their bioavailability (3). Other growth factors are involved in the angiogenesis, notably, PDGFs and their receptors, playing a complementary role to the VEGF family (2).

Thus, VEGF isoforms have been notably associated with poor prognosis in hematologic malignancies such as acute myeloid leukemia (AML) and in many types of solid tumors such as breast cancer. For instance, patent application WO 2005/121362 A2 notably discloses a method for *in vitro* establishing a prognosis concerning a patient having a solid tumor comprising measuring the level of VEGF₁₂₁ and VEGF₁₆₅ transcripts in a biopsy from said tumor, and calculating the VEGF₁₆₅/VEGF₁₂₁ ratio, wherein a VEGF₁₆₅/VEGF₁₂₁ ratio superior to 3 is indicative of a poor prognosis and is therefore indicative if said patient needs an antiangiogenic treatment.

It results that angiogenesis is a privileged target of several agents in the treatment of solid and hematologic malignancies. Among new concepts developed to improve the management of tumors, molecules targeting the VEGF have been developed, and notably molecules inhibiting VEGF receptor (VEGF-R) tyrosine kinase inhibitors (TKI). VEGF-R is indeed a receptor tyrosine kinase (RTK) that has been shown to be not only a key regulator of normal cellular processes but also to have a critical role in the development and progression of many types of cancer. There exists two different VEGF-R binding VEGF at the cell surface, VEGF-R1 (Flt-1) and VEGF-R2 (KDR/Flk-1) possessing a single transmembrane spanning region and an intracellular portion containing a tyrosine-kinase domain. Tyrosine kinases are particularly important today because of their implications in the treatment of cancer. Indeed, phosphorylation of proteins by kinases is an important mechanism in signal transduction for regulation of enzyme activity.

Yet, no predictive factor or biomarker of the response to TKI has been identified. Moreover, there are no clinical factors that can discriminate and/or predict a preferential efficacy of TKI (sunitinib, sorafenib) compared with mTOR inhibitor (temsirolimus), or with bevacizumab (anti-VEGF monoclonal antibody).

For example, sutinitib have proven efficacy in metastatic renal cell carcinoma (mRCC) but the molecular mechanisms underlying the clinical response to these drugs remain unclear. Yet, renal cell carcinoma (RCC) represents 5% of malignancies with 38,000 new cases diagnosed in 2006 in the United States (1) and during last decades, this incidence has constantly increased. At the time of diagnosis, about 30% of RCC are metastatic. For a long time, mRCC has been considered as resistant to the majority of treatments. The management was based on nephrectomy, and the use of immunotherapy (interleukin-2, interferon-α) often poorly tolerated and efficient. Thus, before the introduction of antiangiogenic therapies, the median survival time of mRCC was about twelve months. In first-line therapy, sunitinib significantly improved progression-free survival (PFS) by reducing the risk of relapse of 58% compared with interferon-α (4). However, no predictive clinical or biological factors of response have been identified allowing a better selection of RCC patients for sutitinib therapy.

Currently, only some trends were observed such as slight differences according to age (6), and histological subtypes (7). Interestingly, the occurrence of arterial hypertension has been evoked as a predictive factor of response to sunitinib (8). Faced to this adverse effect, a symptomatic treatment combined with a dose reduction of sunitinib seems a better option than a treatment modification, because of a possible response to sunitinib. An explanation could be the importance of sunitinib metabolism pathway. In terms of biomarkers, a significant correlation has been found between a large decrease in soluble VEGF-R2 and progression-free survival in a small cohort of mRCC treated with sorafenib (9). In a series of five mRCC patients, the free-plasma VEGF has been found to be a possible pharmacodynamic marker for bevacizumab antiangiogenic activity (10). It has been also demonstrated that sunitinib inhibits signaling pathways involved in bevacizumab resistance in mRCC patients, and that baseline levels of soluble VEGF-R3 and VEGF-C may have a potential utility as biomarkers of clinical efficacy in this setting (11). These findings support the potential significance of the VEGF/VEGFR pathway in mRCC, which is predominant. In spite of the inhibition of VEGF-R by sunitinib, the magnitude of its role *in vivo* is not fully clarified.

Indeed, RCC tumor cells, producing high levels of VEGF₁₂₁ and VEGF_{165,} display a higher ability to grow and to invade the extracellular matrix (12, 13). Based on these observations, further researches confirming clinical significance and underlying biologic mechanisms are warranted. Regarding the prognostic value of the VEGF₁₂₁/VEGF₁₆₅ ratio, a predominant expression of VEGF₁₂₁ and VEGF₁₆₅ transcripts has been reported in RCC compared with normal kidney (14, 15). The expression of VEGF mRNA isoforms has been associated with tumor progression and survival in RCC. Indeed, patients with the lower VEGF₁₂₁ mRNA levels had a significantly longer survival compared with those having higher levels. The trend to a lower VEGF₁₂₁ mRNA level in locally advanced RCC indicates that angiogenic activity and degradation might be up-regulated in tumors with a high ability to invade (16). Thereby, patients with high VEGF levels might have a more aggressive disease, and the improved outcome observed in our series might be a reflection of disease biology.

Therefore, if VEGF-R TKI such as sunitinib provide considerable promise for patients, there is a crucial need for a better selection of patients. Indeed, tumors with close characteristics can present opposite behavior with either important and long regressions, or very short-term progressions. Contrary to breast cancer where a target such as HER2 has been discovered prior to anti-HER2 therapies, VEGF-R TKI may also inhibit other tyrosine kinases, which impact other cascades of signaling pathways. Thereby, the optimization of their efficiency is based on a correlation between response to treatment and individual tumor signatures. Indeed, despite recent progress in antitumoral drug development, the treatment of patients affected with a tumor remains a challenge both in term of clinical- and cost-effectiveness. Thus, considering the number of side effects and treatment costs, prediction of antitumoral drug nonresponse early during therapy or even before starting therapy is potentially useful. However, there are no well-established biomarkers, which may improve the selection of patients who may benefit from the treatment.

The purpose of the present invention is therefore to fulfil this need by providing a new reliable method for predicting whether a patient affected with a tumor is responder or no responder to a treatment with a TKI.

### SUMMARY OF THE INVENTION:

The invention relates to a method for predicting the responsiveness of a patient affected with a tumor to a treatment with a tyrosine kinase inhibitor, comprising a step of measuring the expression level of one or two markers selected in the group consisting of isoforms VEGF₁₂₁ or VEGF₁₆₅ in a biological sample from said patient.

In a particular embodiment, the method further comprises a step of comparing the expression level of isoforms VEGF₁₂₁ or VEGF₁₆₅ with control reference values obtained from responder and non-responder group of patients.

The invention also relates to a TKI for treating a patient affected with a tumor, which patient being classified as responder by the method according to the invention.

The invention also relates to the use of VEGF₁₂₁ or VEGF₁₆₅ isoforms as surrogate markers for predicting the responsiveness of a patient affected with a tumor to a treatment with a TKI.

The invention further relates to the use of VEGF₁₂₁/VEGF₁₆₅ ratio for establishing a prognosis and predicting the survival of a patient affected with a renal cell carcinoma (RCC).

### DETAILED DESCRIPTION OF THE INVENTION:

The inventors have shown that the responsiveness of patients affected with a tumor receiving a treatment with a tyrosine kinase inhibitor (TKI) be accurately predicted by determining the expression level of one or two markers selected in the group consisting of isoforms VEGF₁₂₁ or VEGF₁₆₅.

### Definitions:

As used herein, the term "VEGF₁₂₁" has its general meaning in the art and denotes the gene encoding for the VEGF isoform having 121 amino acids after signal sequence cleavage. An exemplary human polynucleotide sequence of VEGF₁₂₁ is disclosed in the International Patent Application WO 2005/131362 as set forth by SEQ ID NO: 24.

As used herein, the term "VEGF₁₆₅" has its general meaning in the art and denotes the gene encoding for the VEGF isoform having 165 amino acids after signal sequence cleavage. An exemplary human polynucleotide sequence of VEGF₁₆₅ is disclosed in the International Patent Application WO 2005/131362 as set forth by SEQ ID NO: 23.

The term "gene" means a DNA sequence that codes for or corresponds to a particular sequence of amino acids which comprise all or part of one or more proteins or enzymes, and may or may not include regulatory DNA sequences, such as promoter sequences, which determine for example the conditions under which the gene is expressed. Some genes, which are not structural genes, may be transcribed from DNA to RNA, but are not translated into an amino acid sequence. Other genes may function as regulators of structural genes or as regulators of DNA transcription. In particular, the term gene may be intended for the genomic sequence encoding a protein, i.e. a sequence comprising regulator, promoter, intron and exon sequences.

The term "expression" when used in the context of expression of a gene or nucleic acid refers to the conversion of the information, contained in a gene, into a gene product. A gene product can be the direct transcriptional product of a gene (e.g., mRNA, tRNA, rRNA, antisense RNA, ribozyme, structural RNA or any other type of RNA) or a protein produced by translation of a mRNA. Gene products also include messenger RNAs which are modified, by processes such as capping, polyadenylation, methylation, and editing, and proteins modified by, for example, methylation, acetylation, phosphorylation, ubiquitination, SUMOylation, ADP-ribosylation, myristilation, and glycosylation.

The term "surrogate marker" is a marker which is differentially expressed in responder patients to treatment with a TKI, such as sunitinib comparatively to non responder patients to the same treatment. Specifically, a surrogate marker may be any gene expression product which is differentially expressed in responder patients when compared to non responder patients. A surrogate marker can be a polynucleotide, a protein, a peptide, or any gene expression product, but is preferably an mRNA or protein expression product. The surrogate markers described herein may also be useful for predicting the responsiveness of a patient affected with a tumor to a treatment with a TKI. Thus, the term "surrogate marker" as used herein refers to those biological molecules which are differentially expressed in response to a treatment with a TKI.

According to the invention, the term "patient", is intended for a human or non-human mammal affected or likely to be affected with a tumor.

A "responder" patient, or group of patients, refers to a patient, or group of patients, who show a clinically significant relief in the disease when treated with a TKI. A contrario, a "non responder patient" or group of patients, refers to a patient or group of patients, who do not show a clinically significant relief in the disease when treated with a TKI. When the disease is metastatic renal cell carcinoma (mRCC), a preferred responder group of patients that provides for the control reference values is a group that shows the disappearance of all target lesions (complete response) or at least 30% decrease in the sum of the longest diameter of such target lesions (partial response) according to RECIST criteria (17, 18), three months after four weeks of treatment with a TKI, preferably sunitinib.

After being tested for responsiveness to a treatment with a TKI, the patients may be prescribed with said TKI.

The terms "tyrosine kinase inhibitor" or " TKI" as used herein refer to any compound, natural or synthetic, which results in a decreased phosphorylation of the tyrosine present on the intracellular domain of receptor tyrosine kinases (RTK) such as growth factor receptors. Accordingly, TKI may be multi-target tyrosine kinase inhibitor and may thus inhibit epidermal growth factor (EGF) receptor family (such as HER-2); insulin-like growth factor (IGF) receptor family (such as IGF-1 receptor); platelet-derived growth factor (PDGF) receptor family, colony stimulating factor (CSF) receptor family (such as CSF-1 receptor); C-Kit receptor and vascular endothelial growth factor (VEGF) receptor family (such as VEGF-R1 (Flt-1) and VEGF-R2 (KDR/Flk-1)).

Preferably, the tyrosine kinase inhibitor is a VEGF-R tyrosine kinase. Typically, the efficacy of the compounds of the invention as inhibitors of VEGF-R tyrosine kinase activity can be demonstrated as described in US patent application 2003/0064992.

### Methods for predicting the responsiveness of a patient according to the invention:

A first aspect of the invention consists of a method for predicting the responsiveness of a patient affected with a tumor to a treatment with a tyrosine kinase inhibitor (TKI), comprising a step of measuring the expression level of one or two markers selected in the group consisting of isoforms VEGF₁₂₁ or VEGF₁₆₅ in a biological sample from said patient.

In a particular embodiment, the method further comprises a step of comparing the expression level of isoforms VEGF₁₂₁ or VEGF₁₆₅ with control reference values obtained from responder and non-responder group of patients.

In a particular embodiment, methods of the invention are suitable for predicting the responsiveness of a patient affected with a tumor to a pharmaceutical treatment with a TKI, wherein said TKI is selected in the group consisting of axitinib, cediranib, dasatinib, nilotinib, pazopanib, semaxanib, sorafenib, sunitinib and vandetanib.

In preferred embodiments, the TKI is sunitinib or N-[2-(diethylamino)ethyl]-5-[(Z)-(5-fluoro-1,2-dihydro-2-oxo-3H-indol-3-ylidine)methyl]-2,4-dimethyl-1H-pyrrole-3 carboxamide (marketed as SUTENT® by Pfizer and previously known as SU11248).

Patients who have been clinically diagnosed as being affected with any tumor are of particular interest in the invention. However in a preferred embodiment the patient is affected with a solid tumor. According to this embodiment, the solid tumor is selected in the group consisting of kidney cancer such as renal cell carcinoma (RCC) and metastatic renal cell carcinoma (mRCC), stomach cancer and gastrointestinal cancer such as gastrointestinal stromal tumor (GIST), hepatic cancer such as hepatocellular carcinoma (HCC), lung cancer, colorectal cancer, breast cancer, head and neck cancer, melanoma, prostate cancer and pancreatic cancer such as pancreatic neuroendocrine tumor.

In one preferred embodiment, the solid tumor is a renal cell carcinoma (RCC) or a metastatic renal cell carcinoma (mRCC).

In another embodiment, the patient is affected with a haematological malignancy. According to this embodiment, the haematological malignancy is selected in the group consisting of multiple myeloma, non-Hodgkin's lymphomas, acute and chronic leukemia (e.g. acute myeloid leukemia (AML).

Samples that may be used for performing the methods according to the invention encompass any biological sample derived from a patient, including any fluids, tissues, cell samples, organs, biopsies, etc.

In a preferred embodiment, the biological sample may result from a resected tumor or a biopsy, and more specifically from a tumor biopsy such as renal tumor biopsy.

Control reference values are easily determinable by the one skilled in the art, by using the same techniques as for determining the level of the same isoforms in biological samples previously collected from the patient under testing.

As indicated above, a first control reference value consists of the mean expression level value of the relevant marker genes that has been determined in a group of patients affected with a tumor that are not responsive to a treatment with a TKI.

A second control reference value consists of the mean expression level value of the relevant marker genes that has been determined in a group of patients affected with a tumor that are responsive to a treatment with a TKI.

At the end of the method according to the invention, a similarity between (i) the expression level value found for the marker genes in the patient tested and (ii) the first control reference value above indicates that the patient tested consists of a non-responder to the treatment with a TKI.

At the end of the method according to the invention, a similarity between (i) the expression level value found for the marker genes in the patient tested and (ii) the second control reference value above indicates that the patient tested consists of a responder to the treatment with a TKI.

Total RNAs or proteins can be easily extracted therefrom. The sample may be treated prior to its use, e.g. in order to render nucleic acids or proteins available. Techniques of cell or protein lysis, concentration or dilution of nucleic acids, are known by the skilled person.

### Determination of the expression level of the marker genes by quantifying mRNAs

Determination of the expression level of a gene such as isoforms VEGF₁₂₁ or VEGF₁₆₅ can be performed by a variety of techniques. Generally, the level as determined is a relative expression level.

More preferably, the determination comprises contacting the sample with selective reagents such as probes, primers or ligands, and thereby detecting the presence, or measuring the amount, of polypeptides or nucleic acids of interest originally in the sample. Contacting may be performed in any suitable device, such as a plate, microtiter dish, test tube, well, glass, column, etc. In specific embodiments, the contacting is performed on a substrate coated with the reagent, such as a nucleic acid array or a specific ligand array. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc.

In a particular embodiment, the expression level may be determined by determining the quantity of mRNA. Such method may be suitable to determine the expression levels of VEGF₁₂₁ or VEGF₁₆₅ genes in a nucleic sample like tumor biopsy.

Methods for determining the quantity of mRNA are well known in the art. For example the nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The extracted mRNA may be then detected by hybridization (e. g., Northern blot analysis).

Alternatively, the extracted mRNA may be subjected to coupled reverse transcription and amplification, such as reverse transcription and amplification by polymerase chain reaction (RT-PCR), using specific oligonucleotide primers that enable amplification of a region in the VEGF₁₂₁ or VEGF₁₆₅ genes. Preferably quantitative or semi-quantitative RT-PCR is preferred. Real-time quantitative or semi-quantitative RT-PCR is particularly advantageous. Extracted mRNA may be reverse-transcribed and amplified, after which amplified sequences may be detected by hybridization with a suitable probe or by direct sequencing, or any other appropriate method known in the art.

Other methods of Amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical.

In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

In a particular embodiment, the method of the invention the steps of providing total RNAs obtained from the biological sample of the patient, and subjecting the RNAs to amplification and hybridization to specific probes, more particularly by means of a quantitative or semi-quantitative RT-PCR.

When quantifying VEGF₁₂₁ or VEGF₁₆₅ transcripts by RT-PCR, according to the invention, the result is preferably expressed as a relative expression of said isoform, having regard to at least one gene with a constant expression level, for example a housekeeping gene such as PPIA (peptidylprolyl isomerase A), TBP (TATA-box binding protein), GADPDH (Glyceraldehyde-3-phosphatedehydrogenase), actine, GUS (beta-glucuronidase), RPLP0 (Ribosomal protein, large, P0) and TFRC (transferrin receptor).

Total RNAs can be easily extracted from a biological sample. For instance, the biological sample may be treated prior to its use, e.g. in order to render nucleic acids available. Techniques of cell or protein lysis, concentration or dilution of nucleic acids, are known by the skilled person.

In another embodiment, the expression level may be determined by DNA microarray analysis. Such DNA microarray or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling. Many variants of the microarray hybridization technology are available to the man skilled in the art

In this context, the invention further provides a DNA microarray comprising a solid support onto which nucleic acids that are specific for VEGF₁₂₁ or VEGF₁₆₅ gene nucleic acid expression products (i.e. mRNA or cDNA) are immobilized.

### Determination of the expression level of the marker genes by quantifying proteins

Other methods for determining the expression level of the said marker genes, namely isoforms VEGF₁₂₁ or VEGF₁₆₅, include the determination of the quantity of polypeptides encoded by the said genes that is found in the sample previously collected from the tumor-affected patient tested. In particular, such methods may be particularly suitable to determine the quantity of VEGF₁₂₁ or VEGF₁₆₅ proteins in a sample like a tumor biopsy.

Such methods comprise contacting a sample with a binding partner capable of selectively interacting with a marker protein present in the sample. The binding partner is generally an antibody that may be polyclonal or monoclonal, preferably monoclonal.

The presence of the protein can be detected using standard electrophoretic and immunodiagnostic techniques, including immunoassays such as competition, direct reaction such as immunohistochemistry, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the antigen and the antibody or antibodies reacted therewith.

The aforementioned assays generally involve separation of unbound protein in a liquid phase from a solid phase support to which antigen-antibody complexes are bound. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads or magnetically responsive beads.

More particularly, an ELISA method can be used, wherein the wells of a microtiter plate are coated with a set of antibodies against the proteins to be tested. A biological sample containing or suspected of containing the marker protein is then added to the coated wells. After a period of incubation sufficient to allow the formation of antibody-antigen complexes, the plate (s) can be washed to remove unbound moieties and a detectably labeled secondary binding molecule added. The secondary binding molecule is allowed to react with any captured sample marker protein, the plate washed and the presence of the secondary binding molecule detected using methods well known in the art.

Alternatively an immunohistochemistry (IHC) method may be preferred. IHC specifically provides a method of detecting targets in a sample or tissue specimen in situ. The overall cellular integrity of the sample is maintained in IHC, thus allowing detection of both the presence and location of the targets of interest. Typically a sample is fixed with formalin, embedded in paraffin and cut into sections for staining and subsequent inspection by light microscopy. Current methods of IHC use either direct labeling or secondary antibody-based or hapten-based labeling. Examples of known IHC systems include, for example, EnVision(TM) (DakoCytomation), Powervision(R) (Immunovision, Springdale, AZ), the NBA(TM) kit (Zymed Laboratories Inc., South San Francisco, CA), HistoFine(R) (Nichirei Corp, Tokyo, Japan).

In particular embodiment, a tissue section (ie sample consisting of cumulus cells) may be mounted on a slide or other support after incubation with antibodies directed against the proteins encoded by the genes of interest. Then, microscopic inspections in the sample mounted on a suitable solid support may be performed. For the production of photomicrographs, sections comprising samples may be mounted on a glass slide or other planar support, to highlight by selective staining the presence of the proteins of interest.

Therefore IHC samples may include, for instance: (a) preparations comprising cumulus cells (b) fixed and embedded said cells and (c) detecting the proteins of interest in said cells samples. In some embodiments, an IHC staining procedure may comprise steps such as: cutting and trimming tissue, fixation, dehydration, paraffin infiltration, cutting in thin sections, mounting onto glass slides, baking, deparaffination, rehydration, antigen retrieval, blocking steps, applying primary antibodies, washing, applying secondary antibodies (optionally coupled to a suitable detectable label), washing, counter staining, and microscopic examination.

### Use of surrogate marker for predicting the responsiveness of a patient:

Another aspect of the invention is the use of VEGF₁₂₁ or VEGF₁₆₅ isoforms as surrogate markers for predicting the responsiveness of a patient affected with a tumor to a treatment with a TKI.

According to this aspect, a significant increase of the expression level of isoforms VEGF₁₂₁ or VEGF₁₆₅ (i.e. at least the doubling of the expression level of isoforms VEGF₁₂₁ or VEGF₁₆₅ comparatively to the expression level of isoforms VEGF₁₂₁ or VEGF₁₆₅ obtained from a non-responder group) is indicative for said patient to be a responder to the treatment with a TKI.

The expression level of VEGF₁₂₁ or VEGF₁₆₅ isoforms may be determined by quantifying mRNAs or by quantifying proteins according to the methods as described above.

In a particular embodiment, the use of VEGF₁₂₁ or VEGF₁₆₅ isoforms as surrogate biomarkers for predicting the responsiveness of a patient affected with a metastatic renal cell carcinoma (mRCC) to a treatment with a TKI.

In another particular embodiment, the use of VEGF₁₂₁ or VEGF₁₆₅ isoforms as surrogate biomarkers for predicting the responsiveness of a patient affected with a tumor to a treatment with sunitinib.

In a preferred embodiment, the use of isoforms VEGF₁₂₁ or VEGF₁₆₅ isoforms as surrogate biomarkers for predicting the responsiveness of a patient affected with a metastatic renal cell carcinoma to a treatment with sunitinib.

### Use of VEGF₁₂₁/VEGF₁₆₅ ratio for establishing a prognosis concerning a patient:

A further aspect of the invention relates to the use of VEGF₁₂₁/VEGF₁₆₅ ratio for establishing a prognosis and predicting the survival of a patient affected with a renal cell carcinoma (RCC).

According to this aspect, a VEGF₁₂₁/VEGF₁₆₅ ratio lower to 1,25 is indicative of a good prognosis for said patient and therefore is indicative for said patient to have a significantly longer survival compared with patient having a ratio higher to 1,25.

In a particular embodiment, the renal cell carcinoma (RCC) is a metastatic renal cell carcinoma (mRCC).

The invention also relates to a method for establishing a prognosis concerning a patient affected by a renal cell carcinoma (RCC) comprising the following steps of:
(a) measuring the expression level of isoforms VEGF₁₂₁ or VEGF₁₆₅ in a biological sample from said patient, and
(b) calculating the VEGF₁₂₁/VEGF₁₆₅ ratio, wherein a VEGF₁₂₁/VEGF₁₆₅ ratio lower to 1,25 is indicative of a good prognosis.

In a particular embodiment, the renal cell carcinoma (RCC) is a metastatic renal cell carcinoma (mRCC).

The expression level of VEGF₁₂₁ or VEGF₁₆₅ isoforms may be determined by quantifying mRNAs or by quantifying proteins according to the methods as described above.

### Therapeutic methods:

A further aspect of the invention relates to a method for the treatment of a tumor in a patient, such as a solid tumor or a hematologic malignancy.

In the context of the invention, the term "treating" or "treatment", as used herein, means reversing, alleviating, inhibiting the progress of, or preventing the disorder or condition to which such term applies, or one or more symptoms of such disorder or condition.

In a particular embodiment, the said method comprises the following steps:
a) determining whether a patient affected with a tumor is a responder or a non responder to a treatment with a TKI, by performing the method as above described; and
b) administering a TKI to said patient, if said patient has been determined as consisting of a responder, at step a) above.

In one preferred embodiment, the TKI is sunitinib.

In another preferred embodiment, the tumor affecting the patient is as renal cell carcinoma (RCC) or a metastatic renal cell carcinoma (mRCC).

A further aspect of the invention is a TKI for treating a patient affected with a tumor, which patient being classified as responder by the method as above described.

The invention will be further illustrated by the following figures and examples. However, these examples and figures should not be interpreted in any way as limiting the scope of the present invention.

### FIGURES:

**Figure 1** is a boxplot of four RNA expression level (in log) standardized with PPIA (peptidylprolyl isomerase A) according to treatment response.
**Figure 2** is a graph representing the overall survival according to the ratio VEGF₁₂₁/VEGF₁₆₅.

### EXAMPLE: ISOFORMS VEGF₁₂₁ or VEGF₁₆₅ LEVEL EXPRESSION IN A RENAL TUMOR BIOPSY OF PATIENTS AFFECTED BY A METASTATIC RENAL CELL CARCINOMA PREDICTS RESPONSIVENESS TO SUNITINIB.

### Material & Methods

**Patients:** We analyzed retrospectively data from 23 consecutive patients with mRCC, and treated in a single-institution (Hôpital Henri Mondor, Créteil, France) with sunitinib after failure of a first-line therapy with interferon-α. Sunitinib was delivered orally at a dose of 50 mg/day for 4 weeks, every 6 weeks. The study was performed in accordance with the Declaration of Helsinki and the International Conference on Harmonization (Good Clinical Practice guidelines).

**Biomarkers evaluated:** A panel of 42 biomarkers was assessed. Two types of biomarkers were analyzed: those involved in angiogenesis, and those involved in invasion, matrix degradation, and migration. According to their expression level, 16 of them were selected for the final analysis. The transcript panel included VEGF (isoforms 121, 165, and 189), and their receptors (VEGF-R1 and -R2); PDGF-A and -B and their receptors (PDGF-Rα and -Rβ); fibroblast growth factor (FGF)-2; HIF-1α; chemokine receptor 4 (CXCR4); uPA, plasminogen activator inhibitor (PAI) -1, PAI-1, and the receptor (uPA-R); and lymphatic vessel endothelial receptor 1 (LYVE-1), which is a transmembrane receptor for the extracellular matrix.

**RNA extraction and quantitative reverse transcription PCR (qRT-PCR):** Total RNA was extracted from frozen renal tissue sections using TRIzol reagent (Invitrogen). First-strand cDNA was synthesized using the High-Capacity cDNA Archive Kit (Applied-Biosystems). Transcript levels were measured in each renal tissue by PCR using Perfect Master Mix-Probe (AnyGenes, France) on LightCycler 2.0 System (Roche). The expression levels of interesting transcripts were normalized to the housekeeping PPIA (peptidylprolyl isomerase A) and TBP (TATA-box binding protein) gene transcripts. Since there was no difference between control genes, results were presented as copies of target gene per copy of PPIA. Gene set assays were designed using Primer-Express Software (Applied-Biosystems) and primers and probes sequences were available upon request. Gene expression levels were determined using standard calibration curves prepared from gene-specific PCR products. All PCRs were done in duplicate.

**Immunohistochemistry:** Immunohistochemical analyses were carried out using antibodies directed against VEGF (Abcam), VEGF-R1, VEGF-R2 (R&D Systems), and PDGF-Rβ (Cell Signaling). Tissue sections were incubated overnight with the specified primary antibody, and then incubated with the appropriate biotinylated secondary antibodies. Peroxidase reactivity was visualized using 3-Amino-9-ethylcarbazole (AEC) (DAKO).

**Statistical Analysis:** Variables analyzed were age at diagnosis, gender, TNM stage, prognosis group at diagnosis according to the Memorial Sloan-Kettering Cancer Center (MSKCC) risk model (5), Fuhrman grade, and treatment response at three months. Gene expression levels were presented in log₁₀ scale. For continuous variables, Mann-Whitney test was used, or Kruskal-Wallis test in the absence of variance equal terms. Gene expression levels between tumoral and non-tumoral tissues were compared by Wilcoxon test for paired data. Survival curves were estimated by the Kaplan-Meier method, and compared using a logrank test. A multivariate analysis was performed using a Cox regression model. All tests and p-value were two-sided, and differences were considered as significant for P < 0.05. Statistical analysis was performed using the Open Source R software (R 2.4.0).

### Results

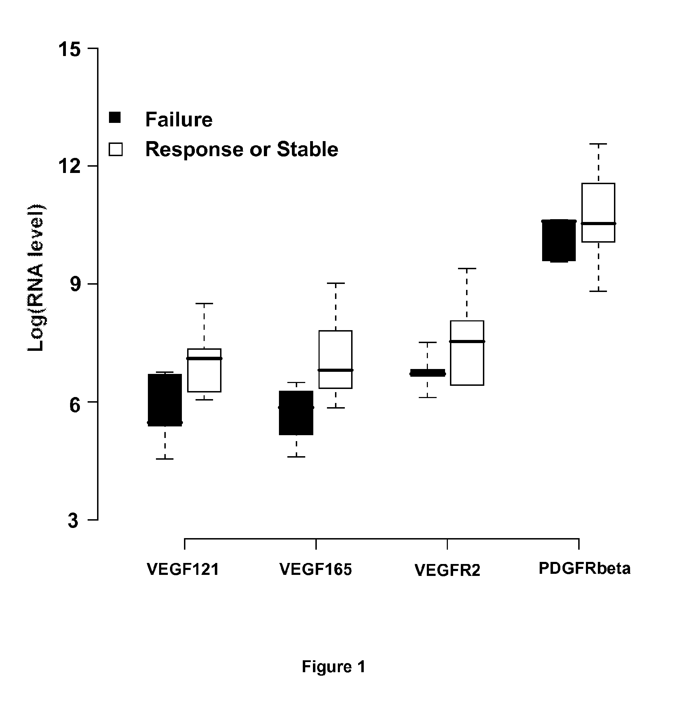

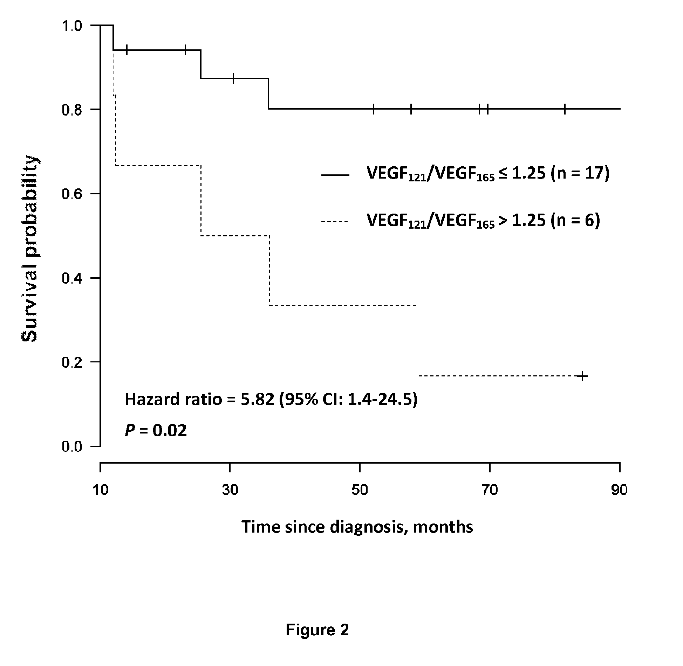

The characteristics of patients are summarized in Table 1. The median age at diagnosis was 56 years (Q1-Q3, 50 to 66 years). Among biomarkers tested, RNA expression levels were significantly different between normal and tumoral tissues for nine of them. A significant increase in VEGF₁₂₁, VEGF₁₆₅, VEGF-R1, PAI-1 and uPA-R was observed in tumoral tissues, whereas there was a significant decrease in PDGF-A, PDGF-B, PDGF-Rβ, and CXCR4. RNA expression levels in tumoral tissues that differed significantly among baseline prognostic factors are presented in Table 2. Transcript levels for VEGF₁₂₁ and VEGF₁₆₅ was significantly higher for the subset of patients with Fuhrman grade 1 or TNM stage 1 tumors. According to RECIST criteria, only VEGF₁₂₁ and VEGF₁₆₅ were significantly associated with the response to sunitinib at three months (Figure 1). The RNA expression level in tumoral tissues was significantly higher in responding patients compared with patients who had a failure to treatment: 1222 versus 241 for VEGF₁₂₁ (P = 0.04), and 905 versus 352 for VEGF₁₆₅ (P = 0.04). For tumors overexpressing VEGF₁₂₁ and VEGF₁₆₅, the probability of response was 81% and 90%, respectively. Additionally, we have explored the corresponding secreted proteins. The immunohistochemical study of total VEGF protein showed a significant correlation between tumor response and difference of VEGF expression between tumor center and margins (P = 0.015). Indeed, higher was this difference, better was the response (data not shown). No significant correlation was found between mRNA levels of VEGF-R1, VEGF-R2 and PDGF receptors (targeted by sunitinib), and response to sunitinib (Figure 1). In this group of 23 patients, the overall survival of patients with an intratumoral VEGF₁₂₁/VEGF₁₆₅ ratio lower than 1.25 (ratio cut-off value determined from the third quartile) was significantly higher than those of patients with a ratio higher than 1.25 (P = 0.02; median survival time, 25.2 months versus not reached; Figure 2). The soluble VEGF isoforms ratio leads to a stronger relationship between VEGF isoforms and survival, and avoids results relative to housekeeping genes.

These findings support the potential significance of the VEGF/VEGFR-2 pathway in mRCC, which is predominant. In spite of the inhibition of VEGF-R2 by sunitinib, the magnitude of its role in vivo is not fully clarified. Clinically, patients who have high levels of VEGF soluble isoforms achieve a better response to sunitinib. Thereby, the VEGF/VEGF-R pathway could be the preferential target of sunitinib. Our results favored the hypothesis that response to therapy is associated with the inhibition of VEGF pathway, depending on the inhibition of VEGF signal. This is likely due to the inhibition of both angiogenesis and cell proliferation driven by the presence of a VEGF/VEGF-R2 autocrine loop in tumor cells. Patients analyzed in this retrospective study presented with more aggressive clear-cell RCC than the usual RCC patients, as more than 60% of them had a histologic grade above 3. In these patients, the response to sunitinib was independent from tumor size, Fuhrman grade, and, noteworthy, from the prognostic group. In this small cohort of mRCC patients, our results suggest that high levels of VEGF₁₂₁ and VEGF₁₆₅ are predictive factors of response to sunitinib.

In conclusion, soluble VEGF isoforms mRNA levels might help the clinician to identify patients who are likely to benefit from TKI such as sunitinib and avoid a costly and potentially toxic administration of this treatment in non-responding patients. This could orientate the therapeutic strategy as patients identified as low responders to sunitinib could receive an alternative treatment.

**Table 1: Patient characteristics**

| **Characteristics** | | **Nr of patients** | **Percentage** |
|---|---|---|---|
| **Gender** | | | |
| | Male | 16 | 69.6 |
| | Female | 7 | 30.4 |

| **TNM stage** | | | |
|---|---|---|---|
| | 1 (T1a/T1b) | 4 | 17.4 |
| | 2 (T2) | 5 | 21.7 |
| | 3 (T3a/T3b) | 14 | 60.9 |

| **Fuhrman grade** | | | |
|---|---|---|---|
| | 1 | 3 | 13.1 |
| | 2 | 5 | 21.7 |
| | 3 | 7 | 30.4 |
| | 4 | 8 | 34.8 |

| **ECOG Performance Status** | | | |
|---|---|---|---|
| | 0 | 19 | 82.6 |
| | 1 | 4 | 17.4 |

| **Sites of metastases*** | | | |
|---|---|---|---|
| | Lung | 15 | 48.4 |
| | Bone | 6 | 19.3 |
| | Lymph nodes | 5 | 16.1 |
| | Other (liver, kidney) | 5 | 16.1 |

| **MSKCC risk factor** | | | |
|---|---|---|---|
| | Favorable | 10 | 43.5 |
| | Intermediate | 11 | 47.8 |
| | Poor | 2 | 8.7 |

| **Treatment response at three months** | | | |
|---|---|---|---|
| | Failure | 5 | 21.7 |
| | Response | 9 | 39.1 |
| | Stabilization | 9 | 39.1 |

| | | | |
|---|---|---|---|
| **Abbreviations:** MSKCC, Memorial Sloan-Kettering Cancer Center; ECOG, Eastern Cooperative Oncology Group * Several sites possible per patient | | | |

**TABLE 2: Statistically Significant RNA Expression Levels in Tumoral Tissues among Baseline Prognostic Factors**

| Factors | | VEGF₁₂₁ | | VEGF₁₆₅ | | VEGF₁₈₉ | | VB5F-R1 | | CXCR4 | | uRA | | PAI-1 | | uPA-R | | LYVE-1 | |
|---|---|---|---|---|---|---|---|---|---|---|---|---|---|---|---|---|---|---|---|
| | | Med | *P* | Med | *P* | Med | *P* | Med | *P* | Med | *P* | Med | *P* | Med | *P* | Med | *P* | Med | *P* |
| Sex | | | | | | | 0.01 | | 0.03 | | | | | | | | 0.01 | | |
| | Male | - | | - | | 0 | | 0.02 | | - | | - | | - | | 2136 | | - | |
| | Female | - | | - | | 0.43 | | 037 | | - | | - | | - | | 1198 | | - | |
| Age | | | | | - | | | | | | | | | | | | 0.04 | | 0.003 |
| | <56yrs | - | | - | | - | | - | | - | | - | | - | | 38 | | 35 | |
| | >56yrs | - | | - | | - | | - | | - | | - | | - | | 12 | | 188 | |
| Fuhrman grade | | | 0.06 | | 0.04 | | | | | | | | 0.02 | | | | | | |
| | 1 | 676 | | 44 | | - | | - | | - | | 0 | | - | | - | | - | |
| | 2 | 48 | | 2 | | - | | - | | - | | 64 | | - | | - | | - | |
| | 3 | 31 | | 1 | | - | | - | | - | | 0 | | - | | - | | - | |
| | 4 | 48 | | 3 | | - | | - | | - | | 0 | | - | | - | | - | |
| Prognosis | | | | | | | | | | | | | | | 0.02 | | | | |
| | Favorable | - | | - | | - | | - | | - | | - | | 10 | | - | | - | |
| | Intermediate | - | | - | | - | | - | | - | | - | | 62 | | - | | - | |
| | Poor | - | | - | | - | | - | | - | | - | | 78 | | - | | - | |
| TNMstage | | | 0.003 | | 0.002 | | | | | | 0.04 | | | | | | | | |
| | 1 | 387 | | 301 | | - | | - | | 274 | | - | | - | | - | | - | |
| | 2 | 105 | | 95 | | - | | - | | 135 | | - | | - | | - | | - | |
| | 3 | 28 | | 21 | | - | | - | | 108 | | - | | - | | - | | - | |

| | | | | | | | | | | | | | | | | | | | |
|---|---|---|---|---|---|---|---|---|---|---|---|---|---|---|---|---|---|---|---|
| Med: median; VEGF: vascular endothelial growth factor; VEGF-R: VEGF receptor; CXCR4: chemokine receptor 4; uPA: urokinase-type plasminogen activator; PAI-1: plasminogen activator inhibitor 1; uPA-R: uPA receptor; LYVE-1: lymphatic vessel endothelial receptor 1. | | | | | | | | | | | | | | | | | | | |

### REFERENCES:

Throughout this application, various references describe the state of the art to which this invention pertains. The disclosures of these references are hereby incorporated by reference into the present disclosure.
1. Jemal A, Siegel R, Ward E, et al. Cancer statistics, 2006. CA Cancer J Clin 2006;56:106-30.
2. Carmeliet P. Angiogenesis in health and disease. Nat Med 2003;9:653-60.
3. Ferrara N, Gerber HP, LeCouter J. The biology of VEGF and its receptors. Nat Med 2003;9:669-76.
4. Motzer RJ, Hutson TE, Tomczak P, et al. Sunitinib versus interferon alfa in metastatic renal-cell carcinoma. N Engl J Med 2007;11:115-24.
5. Motzer RJ, Bacik J, Schwartz LH, et al. Prognostic factors for survival in previously treated patients with metastatic renal cell carcinoma. J Clin Oncol 2004;22:454-63.
6. Eisen T, Oudard S, Szczylik C, et al. Sorafenib for older patients with renal cell carcinoma: subset analysis from a randomized trial. J Natl Cancer Inst 2008;100:1454-63.
7. Choueiri TK, Plantade A, Elson P, et al. Efficacy of sunitinib and sorafenib in metastatic papillary and chromophobe renal cell carcinoma. J Clin Oncol 2008;26:127-31.
8. Rixe O, Billemont B, Izzedine H. Hypertension as a predictive factor of Sunitinib activity. Ann Oncol 2007;18:1117.
9. Escudier B, Szczylik C, Hutson TE, et al. Randomized phase II trial of first-line treatment with sorafenib versus interferon alfa-2a in patients with metastatic renal cell carcinoma. J Clin Oncol 2009;Jan 26.
10. Loupakis F, Falcone A, Masi G, et al. Vascular endothelial growth factor levels in immunodepleted plasma of cancer patients as a possible pharmacodynamic marker for bevacizumab activity. J Clin Oncol 2007;25:1816-8.
11. Rini BI, Michaelson MD, Rosenberg JE, et al. Antitumor activity and biomarker analysis of sunitinib in patients with bevacizumab-refractory metastatic renal cell carcinoma. J Clin Oncol 2008;26:3743-8.
12. Takahashi A, Sasaki H, Kim SJ, et al. Markedly increased amounts of messenger RNAs for vascular endothelial growth factor and placenta growth factor in renal cell carcinoma associated with angiogenesis. Cancer Res 1994;54:4233-7.
13. Tomisawa M, Tokunaga T, Oshika Y, et al. Expression pattern of vascular endothelial growth factor isoform is closely correlated with tumour stage and vascularisation in renal cell carcinoma. Eur J Cancer 1999;35:133-7.
14. Tsuchiya N, Sato K, Akao T, et al. Quantitative analysis of gene expressions of vascular endothelial growth factor-related factors and their receptors in renal cell carcinoma. Tohoku J Exp Med 2001;195:101-13.
15. Rivet J, Mourah S, Murata H, et al. VEGF and VEGFR-1 are coexpressed by epithelial and stromal cells of renal cell carcinoma. Cancer 2008;112:433-42.
16. Ljungberg B, Jacobsen J, Häggström-Rudolfssson S, et al. Tumour vascular endothelial growth factor (VEGF) mRNA in relation to serum VEGF protein levels and tumour progression in human renal cell carcinoma. Urol Res 2003;31:335-40.
17. Therasse P, Arbuck SG, Eisenhauer EA. New guidelines to evaluate the response to treatment in solid tumors. J Natl Cancer Inst. 2000 Feb 2;92(3):205-16.
18. Eisenhauer EA, Therasse P, Bogaerts J, et al. New response evaluation criteria in solid tumours: Revised RECIST guideline (version 1.1). Eur. J. Cancer 2009 Jan;45(2):228-47.

## Claims

1. A method for predicting the responsiveness of a patient affected with a tumor to a treatment with a tyrosine kinase inhibitor (TKI), comprising a step of measuring the expression level of one or two markers selected in the group consisting of isoforms VEGF₁₂₁ or VEGF₁₆₅ in a biological sample from said patient.

2. The method according to claim 1, wherein said method further comprises a step of comparing the expression level of isoforms VEGF₁₂₁ or VEGF₁₆₅ with control reference values obtained from responder and non-responder group of patients.

3. The method according to claim 1 or 2, wherein the TKI is selected in the group consisting of axitinib, cediranib, dasatinib, nilotinib, pazopanib, semaxanib, sorafenib, sunitinib and vandetanib.

4. The method according to claim 3, wherein the TKI is sunitinib.

5. The method according to anyone claims 1 to 4, wherein the tumor is a solid tumor.

6. The method according to claim 5, wherein the solid tumor is selected in the group consisting of kidney cancer such as renal cell carcinoma (RCC), stomach cancer and gastrointestinal cancer such as gastrointestinal stromal tumor (GIST), hepatic cancer such as hepatocellular carcinoma (HCC), breast cancer, lung cancer, colorectal cancer, melanoma, prostate cancer and pancreatic cancer such as pancreatic neuroendocrine tumor.

7. The method according to claim 6, wherein the renal cancer RCC is a metastatic RCC.

8. The method according to anyone claims 1 to 4, wherein the tumor is a haematological malignancy.

9. The method according to claim 8, wherein the haematological malignancy is selected in the group consisting of multiple myeloma, non-Hodgkin's lymphomas, acute and chronic leukemia.

10. The method of any one of claims 1 to 9, wherein the said sample is selected from the group consisting of a resected tumor, a biopsy, a blood sample or a serum sample.

11. The method of any of claims 1 to 10, wherein the level of VEGF₁₂₁ and/or VEGF₁₆₅ is determined by quantifying the level of mRNA of said isoforms in the sample.

12. The method of any of claims 1 to 10, wherein the level of VEGF₁₂₁ and/or VEGF₁₆₅ is determined by quantifying the level of the proteins encoded by said isoforms in the sample.

13. A TKI for treating a patient affected with a tumor, which patient being classified as responder by the method according to any one claims 1 to 12.

14. Use of VEGF₁₂₁ or VEGF₁₆₅ isoforms as surrogate markers for predicting the responsiveness of a patient affected with a tumor to a treatment with a TKI.

15. Use of VEGF₁₂₁/VEGF₁₆₅ ratio for establishing a prognosis and predicting the survival of a patient affected with a renal cell carcinoma (RCC).
